# FASCICULE DE BREVET EUROPEEN

(11) **EP 2 363 078 B1**
(45) Date de publication et mention de la délivrance du brevet: **12.11.2014**
(21) Numéro de dépôt: 11154626.3
(22) Date de dépôt: 16.02.2011
(51) Int. Cl.: A61B 17/04

(54) **Implant de suture**
Naht-Implantat
Suture implant

(30) Priorité: 03.06.2010 FR 1054351; 17.02.2010 US 305464 P
(43) Date de publication de la demande: 07.09.2011
(73) Titulaire: Tornier, Inc., Bloomington, MN 55437 (US)
(72) Inventeur: Koepke, Ryan D., Fort Wayne, IN 46814 (US)
(74) Mandataire: Myon, Gérard Jean-Pierre

(56) Documents cités:
- EP-A2- 2 036 501
- US-A1- 2005 222 619
- US-A1- 2007 112 352
- US-B1- 7 713 285
- US-B2- 6 610 080

## Description

L'invention concerne un implant de suture.

Le domaine de l'invention est celui des implants de suture pour la réparation d'un tissu mou, par exemple un muscle, un tendon ou un ligament devant être fixé à un os.

Lors de certaines blessures, il est fréquent que des ligaments, tendons, et autres tissus mous se détachent des os auxquels ils sont associés. Par conséquent, il devient nécessaire de ré-attacher le tissu mou à l'os afin de faciliter la guérison.

De manière connue, le chirurgien peut disposer de différents dispositifs de ré-attachement du tissu mou à l'os, tel que des vis, agrafes, punaises, clous, ou même un fil de suture seul.

Avantageusement, un implant de suture peut être ancré dans l'os du patient au point désiré de ré-attachement du tissu mou, en permettant la fixation d'un fil de suture chirurgical. Un tel implant de suture peut être inséré dans une cavité osseuse préparée à cet effet, ou être autotaraudeur.

Après insertion dans l'os, l'implant peut être maintenu en position par des éléments externes, par exemple filetage, nervures, ou excroissances de types variés. Ces éléments externes fournissent une résistance à la traction afin que l'implant soit rigidement maintenu en position durant la guérison. Egalement, un tel implant peut comprendre un oeillet, une barre, ou un autre élément pour l'attachement du fil de suture. Ainsi, les extrémités libres de la suture s'étendent à l'extérieur de l'os, passent à travers ou autour du tissu mou, et sont utilisées pour fixer le tissu mou à l'os.

Toutefois, certains implants de suture connus, bien qu'efficaces pour fixer un tissu mou à un os, présentent de nombreux inconvénients.

En pratique, la tête d'implant doit être de dimensions suffisantes pour recevoir un outil d'entraînement, et pour supporter l'effort axial et le couple généré par cet outil d'entraînement lors de l'insertion de l'implant dans l'os. Un tel implant présente une longueur accrue, et doit être positionné dans l'os à une profondeur suffisante pour empêcher le tissu mou de s'écorcher contre la tête d'implant exposée. Cependant, une fois implanté, une tension exercée sur l'implant peut causer sa migration dans l'os et faire sortir la tête d'implant hors de la cavité osseuse. Ce phénomène critique de tension de retrait entraîne un affaiblissement de la fixation et un relâchement des tissus mous, voire même leur endommagement. Par ailleurs, le fil de suture sortant hors de l'implant peut frotter contre l'arête de la cavité osseuse, et être endommagé.

De manière connue, un implant fileté apporte une solution efficace au problème de tension de retrait. Le corps d'un tel implant peut comprendre une boucle ou une barre interne pour l'attachement du fil de suture. Ainsi, le fil de suture est solidement attaché et protégé à l'intérieur du corps de l'implant, mais il ne peut glisser de manière indépendante et sans à-coup. A l'inverse, un glissement du fil de suture peut affecter l'agencement de réparation en modifiant l'interface entre la suture et le tissu, ce qui gêne la guérison. Par ailleurs, lorsque des fils de suture voisins se chevauchent et frottent l'un contre l'autre, ils peuvent être endommagés par friction et abrasion.

US-B-6 610 080 et US-A-2007/0112352 décrivent chacun un implant de suture allongé, comprenant une surface extérieure partiellement filetée, et une partie proximale configurée pour la réception d'au moins un fil de suture. Dans US-B-6 610 080, la partie proximale est traversée par un ou deux oeillets pour le passage de respectivement un ou deux fils de suture. Dans la variante avec deux oeillets transversaux, leurs axes sont sensiblement perpendiculaires à l'axe longitudinal de l'implant de suture, et peuvent être parallèles entre eux, ou bien perpendiculaires entre eux. Dans US-A-2007/0112352, la partie proximale est traversée par deux oeillets qui peuvent être configurés selon différentes variantes. Cependant, le fil de suture est invariablement agencé du côté proximal de l'implant de suture, et n'est donc pas protégé dans le corps de l'implant.

US-A-2005/0222619 décrit un implant de suture allongé, comprenant une surface extérieure entièrement filetée, et une cavité longitudinale qui traverse l'implant d'une extrémité à l'autre et qui est configurée pour la réception d'au moins un fil de suture. Du côté de l'extrémité distale de l'implant, autrement dit sa pointe, la cavité longitudinale est séparée en plusieurs orifices de passage du ou des fils de suture. Toutefois, chaque fil de suture forme un arc à 180° du côté de l'extrémité distale, et n'est donc pas protégé dans le corps de l'implant.

EP-A-2 036 501, sur lequel est basé le préambule de la revendication 1, décrit un implant de suture comprenant un corps allongé muni d'une surface extérieure filetée. Le corps inclut une tige massive cylindrique transverse disposée dans une cavité transversale à l'axe du corps. La surface extérieure de cette tige est rainurée pour délimiter trois chemins de suture, qui sont débouchants vers l'extrémité proximale du corps et non débouchants vers son extrémité distale. Chaque fil de suture en place dans l'implant forme ainsi un arc à 180° autour de la tige, dans la cavité. Cependant, tous les fils de suture traversent cette même cavité, dans laquelle les chemins de suture voisins ne sont que faiblement délimités par les rainures de la tige. De ce fait, un fil de suture peut glisser d'un chemin de suture à un autre et il existe donc des risques de coincement, frottement et usure des fils entre eux. De plus, les rainures sont bordées par des arrêtes vives susceptibles d'endommager les fils de suture en cas de glissement latéral sur la tige.

Le but de la présente invention est de résoudre les différents problèmes susmentionnés : glissement et fixation indépendants de chaque fil de suture, écartement et protection des fils de suture dans le corps de l'implant. En outre, l'implant vise à être compact, simple et économique à fabriquer.

A cet effet, l'invention a pour objet un implant de suture tel que défini à la revendication 1.

Ainsi, l'invention permet d'obtenir un implant de suture amélioré, comprenant des chemins de suture protégés dans le corps de l'implant, qui permet une manipulation aisée et un glissement indépendant des fils de suture. La friction et le risque d'endommagement des fils de suture sont réduits, tout en permettant aux brins de suture d'être alignés dans la même direction. A chaque oeillet correspond un unique chemin de suture, délimité dans le corps de l'implant et la paroi transverse. Ainsi, chaque fil de suture peut glisser dans l'oeillet associé, en formant un arc à 180°, sans risque de chevauchement ou frottement entre arcs voisins et sans risque d'endommagement par des arêtes vives. Par ailleurs, l'agencement des oeillets et de la paroi transverse permet de réaliser un implant compact et simple à fabriquer.

D'autres caractéristiques avantageuses de l'invention, prises isolément ou en combinaison, sont spécifiées aux revendications 2 à 15.

L'invention sera mieux comprise à la lecture de la description qui va suivre, donnée uniquement à titre d'exemple non limitatif et faite en se référant aux dessins sur lesquels :
- la figure 1 est une vue en élévation d'un implant de suture conforme à l'invention,
- la figure 2 est une coupe selon la ligne II-II de la figure 1,
- la figure 3 est une vue en élévation selon la flèche III de la figure 2,
- la figure 4 est une vue en élévation analogue à la figure 1, de l'implant muni des fils de suture,
- la figure 5 est une coupe longitudinale de l'implant, dans un plan parallèle au plan de la figure 4,
- la figure 6 est une coupe selon la ligne VI-VI de la figure 4, et sur laquelle est indiquée la ligne de coupe V-V correspondant à la figure 5,
- la figure 7 est une vue en élévation analogue à la figure 1, d'un deuxième mode de réalisation d'un implant de suture conforme à l'invention,
- la figure 8 est une coupe selon la ligne VIII-VIII de la figure 7,
- la figure 9 est une vue analogue à la figure 7, d'un troisième mode de réalisation d'un implant conforme à l'invention,
- la figure 10 est une coupe selon la ligne X-X de la figure 9,
- la figure 11 est une vue en élévation selon la flèche XI de la figure 10,
- la figure 12 est une coupe analogue à la figure 10, d'un quatrième mode de réalisation d'un implant de suture conforme à l'invention,
- la figure 13 est une vue en élévation selon la flèche XIII de la figure 12,
- la figure 14 est une coupe analogue à la figure 10, d'un cinquième mode de réalisation d'un implant de suture conforme à l'invention,
- la figure 15 est une vue en élévation selon la flèche XV de la figure 14,
- la figure 16 est une vue analogue à la figure 1, d'un sixième mode de réalisation de l'invention,
- la figure 17 est une vue en coupe selon la flèche XVII de la figure 16, et
- la figure 18 est une vue en perspective de l'implant de suture des figures 1 à 6 couplé à un outil d'entraînement.

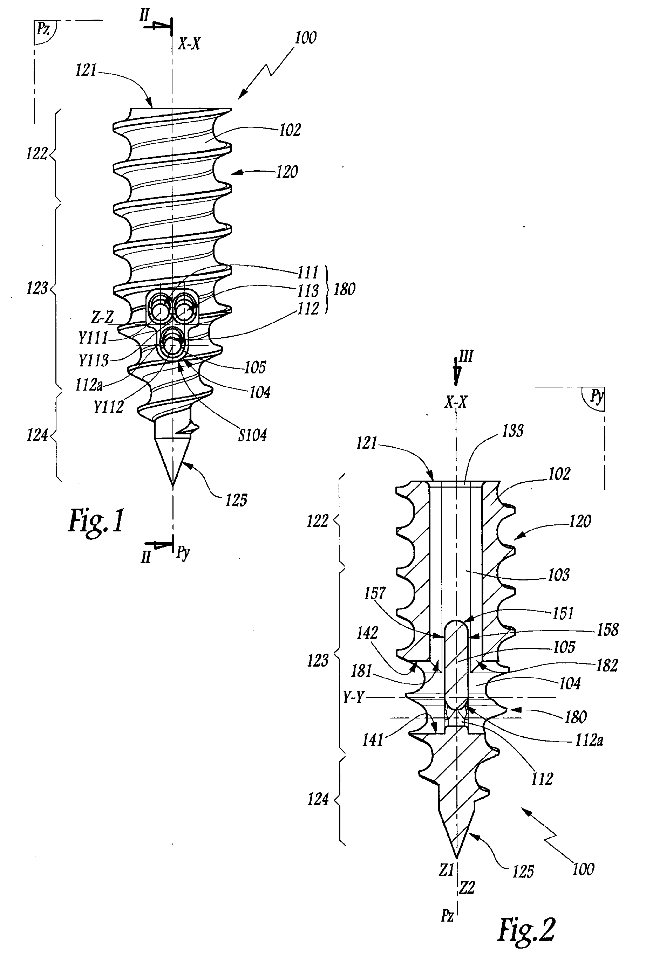

Sur les figures 1 à 6 est représenté un premier mode de réalisation d'un implant de suture 100.

L'implant 100 comprend un corps 102 monobloc et allongé selon un axe central longitudinal X-X. Le corps 102 comprend une surface extérieure 120 entièrement filetée, qui présente un diamètre maximal constant depuis son extrémité proximale 121, sur sa partie proximale 122 et sa partie courante 123, et un diamètre progressivement réduit sur sa partie distale 124 en direction de l'extrémité distale 125, qui est en forme de pointe. Dans le corps 102 sont incluses une cavité proximale 103 et une cavité transversale 104.

Dans le sens de la longueur de l'implant 100, qui est définie par l'axe X-X, la partie courante 123 du corps 102 correspond à la région centrale du corps 102, tandis que la partie proximale 122 est voisine de l'extrémité proximale 121 et que la partie distale 123 est voisine de l'extrémité distale 124. Par exemple, comme visible sur les figures 1, 2 et 6, la partie proximale 122 représente le premier quart de la longueur, la partie courante 123 représente la moitié centrale de la longueur, et la partie distale 124 représente le dernier quart de la longueur du corps 102.

En variante non représentée sur les figures 1 à 6, la partie courante peut s'étendre par exemple sur 2/3 ou bien 3/5 de la longueur du corps.

La cavité proximale 103 s'étend parallèlement à l'axe longitudinal X-X à l'intérieur du corps 102, depuis sa partie courante 123 jusqu'à son extrémité proximale 121 au niveau de laquelle la cavité proximale 103 débouche sur l'extérieur. Sur les figures 1 à 6, la cavité proximale 103 présente une section transversale de forme sensiblement carrée avec des angles arrondis, et une symétrie axiale par rapport à l'axe longitudinal X-X. Du côté de l'extrémité proximale 121, la bordure 133 de la cavité proximale 103 présente une forme sensiblement arrondie vers l'extérieur et aucune arête vive.

En variante non représentée sur les figures 1 à 6, la section transversale de la cavité proximale 103 est hexagonale. En effet, la fonction de la cavité 103 est de réaliser l'interface entre l'implant 100 et la pointe d'un outil, et celle-ci peut donc présenter différentes formes de section.

La cavité transversale 104 traverse le corps 102 dans sa partie courante 123, en s'étendant selon un premier axe transversal Y-Y qui est sensiblement perpendiculaire à l'axe longitudinal X-X. Sa section S104 considérée dans un plan perpendiculaire à l'axe Y-Y est sensiblement identique le long de l'axe Y-Y. La cavité proximale 103 débouche dans la cavité transversale 104, au niveau d'une face proximale 142 de la cavité transversale 104.

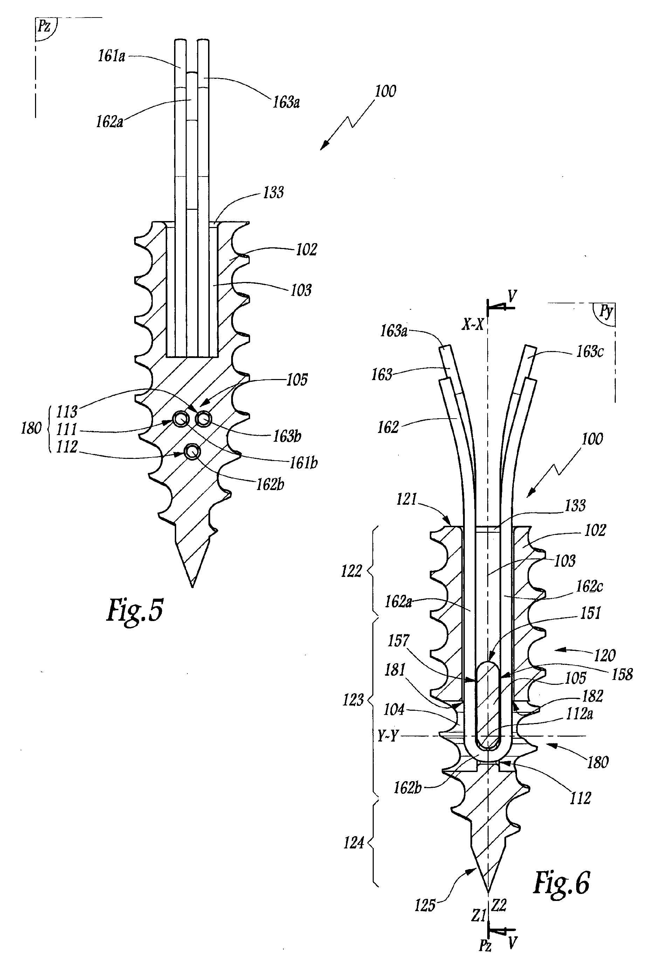

Une paroi transverse 105 est agencée dans la cavité transversale 104, au niveau de la partie courante 123 du corps 102. La paroi 105 s'étend en direction de la cavité proximale 103 depuis une face distale 141 de la cavité transversale 104, et s'étend partiellement à l'intérieur de la cavité proximale 103, en se terminant par une extrémité 151 de forme arrondie. La paroi 105 présente un plan de symétrie Pz, comprenant l'axe longitudinal X-X et un deuxième axe transversal Z-Z qui est perpendiculaire aux axes X-X et Y-Y. Ainsi, le plan Pz délimite un premier côté Z1 et un second côté Z2, comme visible sur les figures 2, 3 et 6. La paroi 105 partage la cavité transversale 104 en deux parties de cavité situées de chaque côté Z1 ou Z2 du plan Pz. La paroi 105 s'étend essentiellement dans le plan Pz et présente deux faces 157 et 158 sensiblement planes, respectivement du côté Z1 et du côté Z2. Ces faces 157 et 158 sont parallèles entre elles et perpendiculaires au premier axe Y-Y. Les dimensions de la paroi 105 sont plus réduites dans la direction définie par cet axe Y-Y que dans les directions définies par les axes X-X et Z-Z. A titre d'exemple montré aux figures 2 et 6, la longueur de la paroi 105 suivant l'axe X-X est au moins quatre fois plus grande que la largeur de cette paroi 105 suivant l'axe Y-Y. Ainsi, la paroi 105 peut être qualifiée de paroi mince.

On définit un plan Py, qui comprend l'axe longitudinal X-X et le premier axe transversal Y-Y, et qui est donc perpendiculaire au plan Pz. Ainsi, en excluant la surface extérieure filetée 120, les plans Py et Pz sont des plans de symétrie de l'implant 100.

Dans la paroi 105 sont agencés trois oeillets traversants 111, 112 et 113, qui s'étendent entre les faces 157 et 158 sensiblement parallèlement à l'axe Y-Y selon des axes respectifs Y111, Y112 et Y113, lesquels sont donc perpendiculaires au plan Pz. L'axe Y112 de l'oeillet 112 est sécant avec l'axe X-X, tandis que les axes Y111 et Y113 des oeillets correspondants 111 et 113 sont situés de part et d'autre de l'axe X-X. Ainsi, chaque oeillet 111, 112 et 113 traverse le plan Pz et relie les deux côtés Z1 et Z2. En d'autres termes, chaque oeillet 111, 112 et 113 traverse la paroi 105 et débouche au niveau des faces 157 et 158.

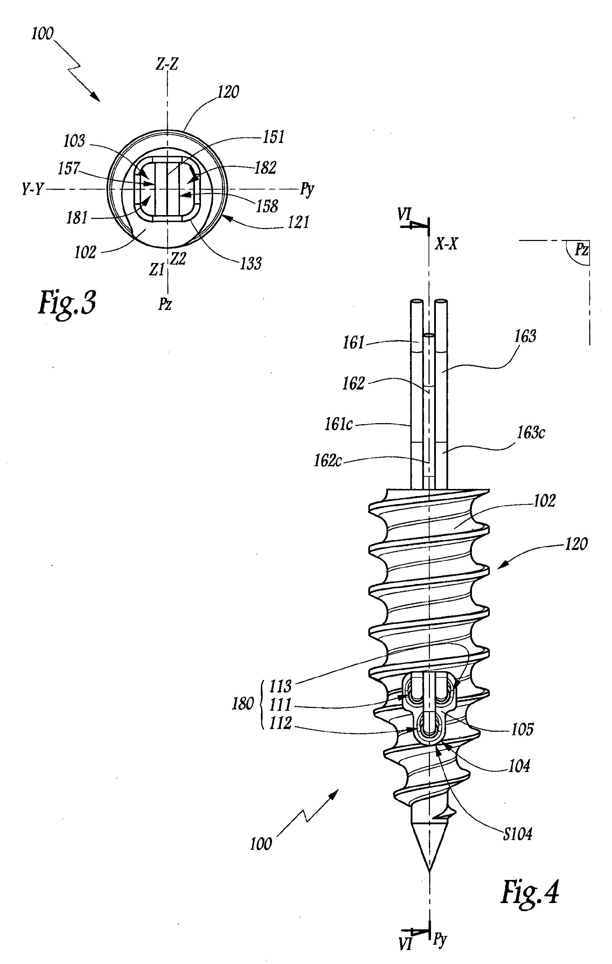

Comme visible sur les figures 4 à 6, plusieurs fils de suture 161, 162, 163 peuvent être agencés dans le corps 102 de l'implant 100, plus précisément dans des chemins de suture respectifs. En position de service, chaque fil 161, 162 ou 163 comprend deux brins sensiblement rectilignes et parallèles, respectivement un premier brin 161 a, 162a ou 163a situé du côté Z1 et un second brin 161c, 162c ou 163c situé du côté Z2, ainsi qu'un arc 161b, 162b ou 163b formé dans l'oeillet correspondant 111, 112 ou 113 et qui relie l'un à l'autre les deux brins précités.

Lors du positionnement de chaque fil de suture 161, 162 ou 163 dans son chemin de suture propre situé dans l'implant 100, le premier brin 161 a, 162a ou 163a pénètre dans le corps 102 au niveau de l'extrémité proximale 121, puis traverse la cavité proximale 103 et la cavité transversale 104 en étant sensiblement parallèle à l'axe longitudinal X-X. Ensuite, dans la partie courante 123 du corps 102, le fil 161, 162 ou 163 traverse la paroi 105 en formant à 180° l'arc 161b, 162b ou 163b dans l'oeillet correspondant 111, 112 ou 113, dans lequel il peut glisser librement. Puis le fil 161, 162 ou 163 traverse en sens inverse la cavité transversale 104 et la cavité proximale 103, sous la forme du second brin 161c, 162c ou 163c sensiblement parallèle au premier brin 161 a, 162a ou 163a, pour ressortir du corps 102 au niveau de l'extrémité proximale 121. Dans la partie de liaison avec l'arc 161 b, 162b ou 163b associé, le brin rectiligne 161 a, 162a ou 163a longe la face 157, tandis que le brin rectiligne 161c, 162c ou 163c longe la face 158. En outre, le premier brin 161 a, 162a ou 163a traverse la cavité proximale 103 puis la cavité transversale 104 en étant positionné sensiblement du côté Z1, tandis que le second brin 161c, 162c ou 163c traverse la cavité transversale 104 puis la cavité proximale 103 en étant positionné sensiblement du côté Z2.

Ainsi, l'implant 100 comprend trois chemins de suture, qui correspondent respectivement aux oeillets distincts 111, 112 et 113 et permettent le passage des fils 161, 162 et 163. Les chemins de suture sont débouchants vers l'extrémité proximale 121 et non débouchants vers l'extrémité distale 125. Chaque chemin de suture présente une partie 181 située du côté Z1 pour le passage du premier brin, une partie 182 située du côté Z2 pour le passage du second brin, et un espace transversal 180 qui traverse le plan Pz et dans lequel est agencé l'arc du fil de suture. La cavité proximale 103, la cavité transversale 104 et la paroi transverse 105 délimitent conjointement les chemins de suture, avec les oeillets 111, 112 et 113. L'espace transversal 180 est apte à recevoir au moins un fil de suture et est avantageusement situé dans la partie courante 123 du corps 102. Autrement dit, dans le premier mode de réalisation, les oeillets 111, 112 et 113 délimitent l'espace transversal 180 des chemins de suture, tandis que les parties latérales 181 et 182 des chemins de suture s'étendent depuis les oeillets de part et d'autre de la paroi transverse 105, le long des faces 157 et 158, en direction de l'extrémité proximale 121. La paroi 105 peut donc être qualifiée de paroi de séparation entre les parties latérales 181 et 182 des chemins de suture. Au niveau de cette paroi 105, les brins rectilignes 161 a, 162a, 163a, 161c, 162c et 163c s'étendent le long des faces 157 et 158 respectives, en direction de la paroi proximale 103 et de l'extrémité proximale 121.

A ce stade, on remarque que les deux oeillets 111 et 113 sont décalés axialement, c'est-à-dire selon la direction définie par l'axe longitudinal X-X, dans la paroi transverse 105 par rapport à l'oeillet 112. En outre, les deux oeillets 111 et 113 sont décalés transversalement entre eux, selon la direction définie par l'axe Z-Z, dans la paroi transverse 105. En d'autres termes, les axes Y111, Y112 et Y113 des trois oeillets 111, 112 et 113 associés aux trois chemins de suture définissent un triangle isocèle dans le plan Pz. Ainsi, les oeillets 111, 112 et 113 sont agencés dans la paroi 105 en suivant un compromis entre écartement des chemins de suture et compacité de l'implant 100.

En pratique, l'écartement transversal des oeillets 111, 112 et 113 permet d'éviter que les brins de suture 161 a, 162a, 163a, 161c, 162c et 163c se croisent ou se chevauchent, et ainsi frottent les uns contre les autres. Ainsi, ils ne s'usent pas sous l'effet de la friction ou de l'abrasion, tout en étant alignés sensiblement dans la même direction. Comme l'arc 161b, 162b, 163b de chacun des fils de suture 161, 162, 163 est agencé à l'intérieur du corps 102 de l'implant 100, dans sa partie courante 123, il est mieux protégé que s'il était disposé à l'une ou l'autre extrémité 121 ou 125 du corps 102 de l'implant 100.

Comme visible sur les figures 2 et 6, la face proximale 112a de l'oeillet 112, située du côté de la cavité proximale 103, est de forme convexe sensiblement parabolique. Il en est de même pour les faces proximales des oeillets 111 et 113, non représentées. De plus, les bordures des oeillets présentent une forme sensiblement arrondie et aucune arête vive. Ainsi, lorsque le fil de suture 161, 162 ou 163 forme l'arc 161 b, 162b ou 163b à 180° dans l'oeillet de passage correspondant 111, 112 ou 113, il peut s'appuyer sur la face proximale de l'oeillet, dans lequel il peut glisser sans être endommagé par des arêtes vives.

Par ailleurs, la cavité transversale 104 s'étend du côté de la partie courante 123 qui est plus proche de la partie distale 124 que de la partie proximale 122, en débouchant transversalement de part et d'autre du corps 102. De ce fait, la cavité transversale 104 et la paroi 105, et donc l'espace transversal 180 de passage des fils 161, 162 et 163, sont suffisamment éloignés de l'extrémité distale 125 pour protéger efficacement les fils 161, 162 et 163.

En outre, chaque fil 161, 162 ou 163 n'est pas maintenu rigidement en position dans l'implant 100. Au contraire, le glissement permet de faciliter la réalisation de noeuds par le chirurgien à l'extérieur de l'implant 100.

Dans ce premier mode de réalisation, le profil de la section S104 de la cavité transversale 104, définie plus haut, correspond sensiblement à l'agencement des trois oeillets 111, 112 et 113, notamment en suivant un profil courbe, sans angles vifs. Les formes arrondies sont plus simples à façonner dans le corps 102 de l'implant, par exemple par usinage, notamment perçage ou fraisage, ou par moulage.

En variante non représentée, la section S104 peut être de forme sensiblement rectangulaire, elliptique ou triangulaire, notamment avec des angles arrondis.

De préférence, le corps 102 de l'implant de suture 100 est réalisé en un matériau métallique, notamment titane. L'implant 100 en titane présente une résistance et des propriétés de biocompatibilité importantes, et est également muni d'une pointe qui facilite son insertion directement dans l'os.

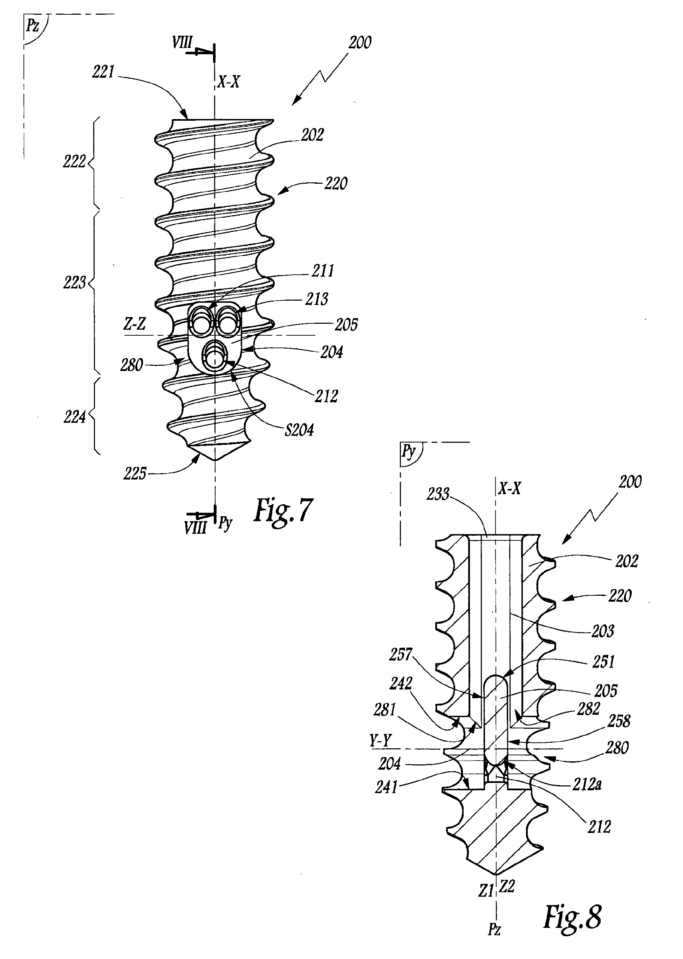

Sur les figures 7 et 8 est représenté un implant de suture 200.

Certains éléments constitutifs de l'implant 200 sont similaires aux éléments constitutifs de l'implant 100 du premier mode de réalisation, décrit plus haut, et portent la même référence augmentée de cent. Il s'agit de la surface extérieure filetée 220 du corps 202, de l'extrémité proximale 221, de la partie proximale 222, de la partie courante 223, de la partie distale 224, de l'extrémité distale 225, de la cavité proximale 203, des faces respectivement distale 241 et proximale 242 de la cavité transversale 204, de l'extrémité arrondie 251 de la paroi transverse 205, des faces 257 et 258, des oeillets 211, 212 et 213, et de la face proximale parabolique 212a de l'oeillet 212. Les axes des oeillets 211, 212 et 213 ne sont pas représentés dans un but de simplification.

Dans ce deuxième mode de réalisation, l'implant 200 comprend trois chemins de suture qui correspondent respectivement aux oeillets 211, 212 et 213. Chaque chemin de suture est débouchant vers l'extrémité proximale 221 et non débouchant vers l'extrémité distale 225. Les oeillets 211, 212 et 213 délimitent l'espace transversal 280 des chemins de suture, tandis que les parties latérales 281 et 282 des chemins de suture s'étendent depuis les oeillets de part et d'autre de la paroi transverse 205, en direction de l'extrémité proximale 221, respectivement du côté Z1 et du côté Z2. La paroi 205 s'étend essentiellement dans le plan Pz, tandis que les deux faces 257 et 258 sont sensiblement planes et perpendiculaires à l'axe Y-Y. Les axes des trois oeillets 211, 212 et 213 associés aux trois chemins de suture définissent un triangle isocèle dans le plan Pz.

De préférence, l'implant de suture 200 est réalisé en un matériau polymère, notamment polyétheréthercétone (PEEK). L'implant 200 en PEEK présente des propriétés de biocompatibilité et de radiotransparence importantes, et peut être percé facilement lors d'une révision, toutefois son implantation nécessite la formation préalable d'une cavité osseuse. En effet, par comparaison avec l'implant 100, la réduction de diamètre dans la partie distale 224 est moins prononcée, et l'extrémité distale 225 du corps 202 ne présente pas une pointe distincte de la partie distale 204.

En outre, comme visible sur la figure 7, la section S204 de la cavité transversale 204 considérée dans un plan perpendiculaire à l'axe transversal Y-Y est légèrement différente. La section S204 présente un profil qui correspond sensiblement à l'agencement des trois oeillets 211, 212 et 213, avec une partie de profil proximale de forme sensiblement rectangulaire aux angles arrondis, et une partie de profil distale de forme arrondie, sans angle aigu. En pratique, le profil de la section S204 est plus simple à réaliser que le profil de la section S104 de l'implant 100, mais l'une ou l'autre variante convient à un implant de suture conforme à l'invention.

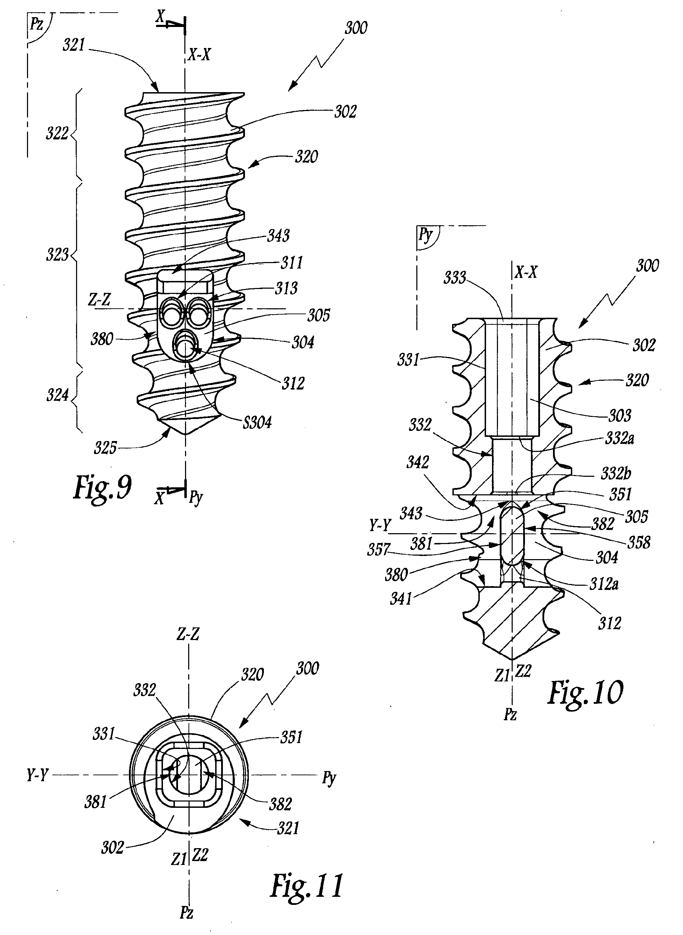

Sur les figures 9, 10 et 11 est représenté un implant de suture 300.

Certains éléments constitutifs de l'implant 300 sont similaires aux éléments constitutifs de l'implant 200 du premier mode de réalisation, décrit plus haut, et portent la même référence augmentée de cent. Il s'agit de la surface extérieure filetée 320 du corps 302, de l'extrémité proximale 321, de la partie proximale 322, de la partie courante 323, de la partie distale 324, de l'extrémité distale 325, des faces respectivement distale 341 et proximale 342 de la cavité transversale 304, de l'extrémité arrondie 351 de la paroi transverse 305, des faces 357 et 358, des oeillets 311, 312 et 313, et de la face proximale parabolique 312a de l'oeillet 312.

Dans ce troisième mode de réalisation, l'implant 300 comprend trois chemins de suture qui correspondent respectivement aux oeillets 311, 312 et 313. Chaque chemin de suture est débouchant vers l'extrémité proximale 321 et non débouchant vers l'extrémité distale 325. Les oeillets 311, 312 et 313 délimitent l'espace transversal 380 des chemins de suture, tandis que les parties latérales 381 et 382 des chemins de suture s'étendent depuis les oeillets de part et d'autre de la paroi transverse 305, en direction de l'extrémité proximale 321, respectivement du côté Z1 et du côté Z2. La paroi 305 s'étend essentiellement dans le plan Pz, tandis que les deux faces 357 et 358 sont sensiblement planes et perpendiculaires à l'axe Y-Y. A titre d'exemple montré à la figure 10, la longueur de la paroi 305 suivant l'axe X-X est près de trois fois plus grande que la largeur de cette paroi 305 suivant l'axe Y-Y. Les axes des trois oeillets 311, 312 et 313 associés aux trois chemins de suture définissent un triangle isocèle dans le plan Pz.

Comme visible sur la figure 10, la cavité proximale 303 comprend deux portions distinctes 331 et 332 s'étendant selon l'axe longitudinal X-X du corps 302 de l'implant 300. La première portion de cavité 331 est agencée du côté de l'extrémité proximale 321, et présente une section transversale de forme sensiblement carrée, avec des angles arrondis. La deuxième portion de cavité 332 est agencée entre cette première portion de cavité 331 et la cavité transversale 304, et présente une section transversale de forme sensiblement circulaire. Chacune des extrémités longitudinales 332a et 332b de la deuxième portion de cavité 332 présente une bordure chanfreinée, en particulier de forme sensiblement arrondie et sans aucune arête vive, pour ne pas endommager les fils de suture 361, 362 et 363 lors de leur passage.

La section S304 de la cavité transversale 304 est plus grande que la section S204 de l'implant 200. En outre, à la différence des implants 100 et 200, le corps 302 comprend dans sa partie courante 323 un orifice transverse traversant 343 autre que les oeillets 311, 312 et 313 traversant la paroi transverse 305. Cet orifice 343 facilite la fabrication de l'implant 300, en permettant notamment d'utiliser un foret de dimensions plus importantes pour réaliser une canulation dans le corps 302.

La paroi transverse 305 est agencée à l'intérieur de la cavité transversale 304 et ne s'étend pas, même partiellement, dans la cavité proximale 303. Du côté orienté vers l'extrémité proximale 321, l'extrémité 351 de la paroi transverse 305 est arrondie de manière convexe dans le plan Py, et de manière concave dans le plan Pz, afin de ne présenter aucune arête vive susceptible d'endommager les fils de suture.

Lors du positionnement des fils de suture dans l'implant 300, tels que les fils 161, 162 et 163 évoqués plus haut en relation avec l'implant 100, un premier brin de chaque fil pénètre dans le corps 302 au niveau de l'extrémité proximale 321, puis traverse la première portion 331 et la seconde portion 332 de la cavité proximale 303 et la cavité transversale 304 en étant sensiblement parallèle à l'axe longitudinal X-X. Ensuite, dans la partie courante 323 du corps 302, chaque fil traverse la paroi 305 en formant un arc à 180° dans l'oeillet correspondant 311, 312 ou 313, dans lequel il peut glisser librement. Puis chaque fil traverse en sens inverse la cavité transversale 304 et la première portion 331 et la seconde portion 332 de la cavité proximale 303, sous la forme d'un second brin sensiblement parallèle au premier brin précité, pour ressortir du corps 302 au niveau de l'extrémité proximale 321. Ainsi, ces fils de suture sont regroupés dans la seconde portion 332 de la cavité proximale 303, sans toutefois se chevaucher et frotter excessivement les uns contre les autres.

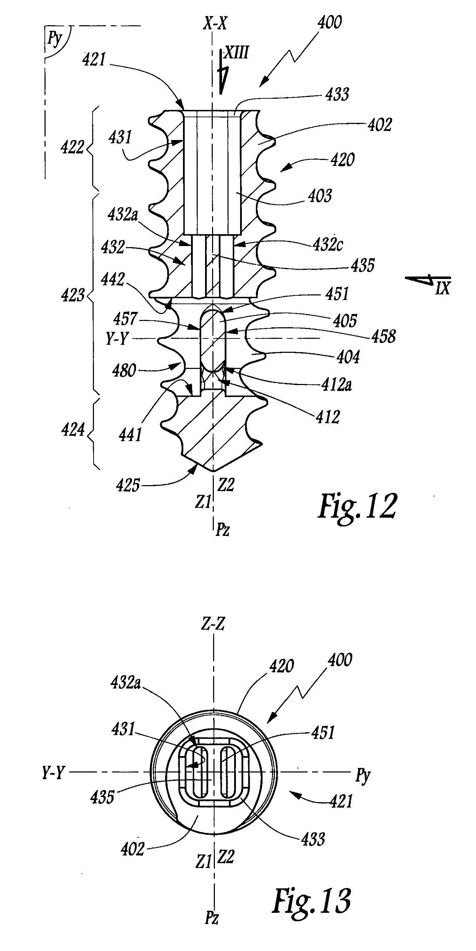

Sur les figures 12 et 13 est représenté un implant de suture 400.

Certains éléments constitutifs de l'implant 400 sont similaires aux éléments constitutifs de l'implant 300 du troisième mode de réalisation, décrit plus haut, et portent la même référence augmentée de cent. Il s'agit de la surface extérieure filetée 420 du corps 402, de l'extrémité proximale 421, de la partie proximale 422, de la partie courante 423, de la partie distale 424, de l'extrémité distale 425, des faces respectivement distale 441 et proximale 442 de la cavité transversale 404, de la section S404, de l'extrémité arrondie 451 de la paroi transverse 405, des faces 457 et 458, des oeillets 411, 412 et 413, et de la face proximale parabolique 412a de l'oeillet 412.

Si on observe l'implant 400 selon la flèche IX à la figure 12, on retrouve une vue en élévation analogue à la figure 9.

En comparaison avec l'implant 300, la cavité proximale 403 de l'implant 400 comprend également deux portions de cavité 431 et 432 distinctes, mais la deuxième portion de cavité 432 comprend deux orifices 432a et 432c séparés par une paroi 435. Chacun des orifices 432a et 432c présente une section transversale de forme sensiblement rectangulaire, dont les extrémités larges parallèles à l'axe Z-Z sont linéaires, et dont les extrémités étroites sont arrondies. Les parois longitudinales de plus grandes dimensions des orifices 432a et 432c sont parallèles au plan Pz, et donc parallèles aux faces longitudinales de la paroi transverse 405 qui sont parallèles au plan Pz. Autrement dit, la paroi de séparation 435 s'étend parallèlement au plan Pz. Le premier orifice 432a est destiné à recevoir du côté Z1 le premier brin de chacun des fils de suture, tandis que le second orifice 432c est destiné à recevoir du côté Z2 le second brin de chacun des fils de suture, non représentés.

En pratique, les premiers brins sont regroupés entre eux et les seconds brins sont regroupés entre eux, mais les premiers sont séparés des seconds dans leur orifice respectif 432a ou 432c, situés de part et d'autre de la paroi 435. Ainsi, le risque de chevauchement et de friction, et donc d'endommagement des fils de suture, est plus limité.

Ainsi, l'implant 400 comprend trois chemins de suture qui correspondent respectivement aux oeillets 411, 412 et 413. Chaque chemin de suture est débouchant vers l'extrémité proximale 421 et non débouchant vers l'extrémité distale 425. Dans ce quatrième mode de réalisation, les oeillets 411, 412 et 413 délimitent l'espace transversal 480, tandis que les orifices 432a et 432c constituent les parties latérales des chemins de suture, débouchantes vers l'extrémité proximale 421 et non débouchantes vers l'extrémité distale 425. La paroi 405 s'étend essentiellement dans le plan Pz, tandis que les deux faces 457 et 458 sont sensiblement planes et perpendiculaires à l'axe Y-Y. Les axes des trois oeillets 411, 412 et 413 associés aux trois chemins de suture définissent un triangle isocèle dans le plan Pz.

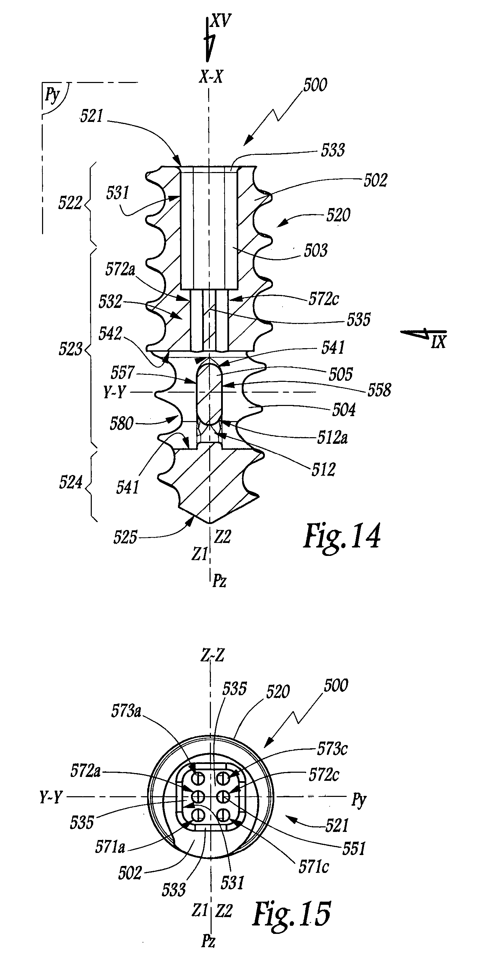

Sur les figures 14 et 15 est représenté un implant de suture 500.

Certains éléments constitutifs de l'implant 500 sont similaires aux éléments constitutifs de l'implant 400 du quatrième mode de réalisation, décrit plus haut, et portent la même référence augmentée de cent. Il s'agit de la surface extérieure filetée 520 du corps 502, de l'extrémité proximale 521, de la partie proximale 522, de la partie courante 523, de la partie distale 524, de l'extrémité distale 525, des faces respectivement distale 541 et proximale 542 de la cavité transversale 504, de la section S504, de l'extrémité arrondie 551 de la paroi transverse 505, des faces 557 et 558, des oeillets 511, 512 et 513, et de la face proximale parabolique 512a de l'oeillet 512.

Si on observe l'implant 500 selon la flèche IX à la figure 14, on retrouve une vue en élévation analogue à la figure 9.

En comparaison avec les implants 300 et 400, la cavité proximale 503 de l'implant 500 comprend également deux portions de cavité 531 et 532 distinctes, mais la deuxième portion de cavité 532 comprend six orifices longitudinaux 571 a, 572a, 573a, 571c, 572c et 573c, qui s'étendent parallèlement à l'axe longitudinal X-X. Chacun des orifices présente une section transversale de forme circulaire, de diamètre légèrement supérieur au diamètre des fils de suture, non représentés. A chaque oeillet correspondent deux orifices, qui sont agencés dans la seconde portion 532 de la cavité proximale 503 de manière symétrique par rapport au plan Pz, et dont les axes respectifs sont sécants avec l'axe de l'oeillet correspondant. Le premier orifice est destiné à recevoir le premier brin du fil de suture correspondant, tandis que le second orifice est destiné à recevoir le second brin du fil de suture correspondant, non représenté.

En pratique, l'implant 500 comprend trois chemins de suture qui correspondent respectivement aux oeillets 511, 512 et 513. Chaque chemin de suture est débouchant vers l'extrémité proximale 521 et non débouchant vers l'extrémité distale 525. Chaque brin de chaque fil de suture dispose de son propre orifice de passage 571 a, 572a, 573a, 571c, 572c ou 573c dans la cavité proximale 503, et peut suivre son propre chemin de suture sans contact avec les autres fils dans la portion de cavité 532. Ainsi, le risque de chevauchement et de friction, et donc d'endommagement des fils de suture, est encore plus limité que pour les implants 300 et 400.

La paroi 505 s'étend essentiellement dans le plan Pz, tandis que les deux faces 557 et 558 sont sensiblement planes et perpendiculaires à l'axe Y-Y. Les axes des trois oeillets 511, 512 et 513 associés aux trois chemins de suture définissent un triangle isocèle dans le plan Pz.

Par ailleurs, on définit une paroi de séparation 535, visible sur les figures 14 et 15, qui est située dans la portion de cavité 532, qui s'étend parallèlement au plan Pz, et qui sépare les trois orifices 571 a, 572a et 573a situés du côté Z1 des trois orifices 571c, 572c et 573c qui sont situés du côté Z2. Dans ce cinquième mode de réalisation, les oeillets 511, 512 et 513 délimitent l'espace transversal 580, tandis que les orifices 571 a, 572a, 573a, 571c, 572c et 573c constituent les parties latérales des chemins de suture, débouchantes vers l'extrémité proximale 521 et non débouchantes vers l'extrémité distale 525.

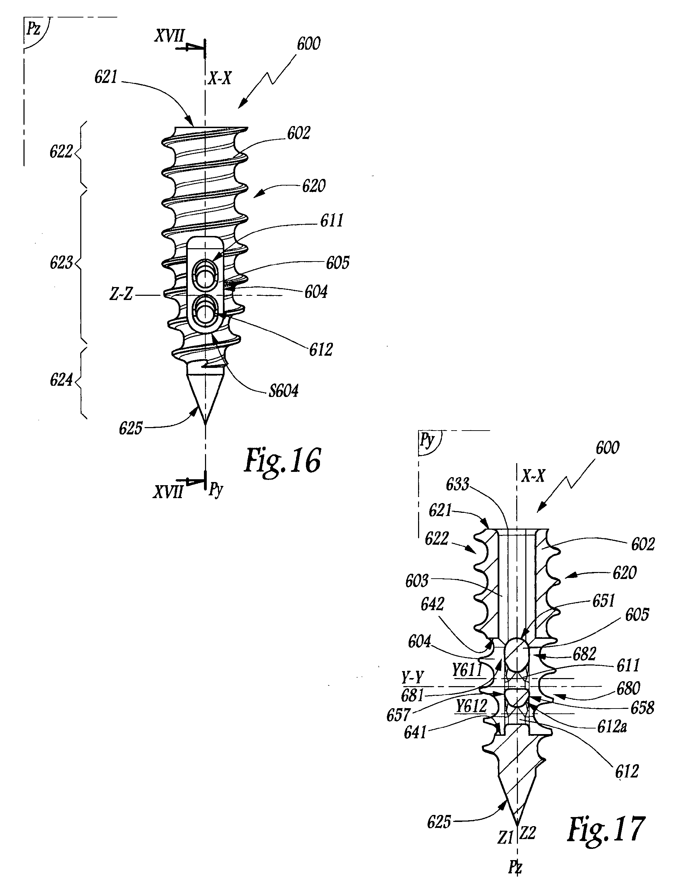

Sur les figures 16 est 17 est représenté un implant de suture 600.

Certains éléments constitutifs de l'implant 600 sont similaires aux éléments constitutifs de l'implant 100 du premier mode de réalisation, décrit plus haut, et portent la même référence augmentée de cinq cent. Il s'agit de la surface extérieure filetée 620 du corps 602, de l'extrémité proximale 621, de la partie proximale 622, de la partie courante 623, de la partie distale 624, de l'extrémité distale 625, de la cavité proximale 603, des faces respectivement distale 641 et proximale 642 de la cavité transversale 604, de la section S604, de l'extrémité arrondie 651 de la paroi transverse 605, des faces 657 et 658, et de la face proximale parabolique 612a de l'oeillet 612.

Le corps 602 de l'implant 600 présente un diamètre plus réduit que dans les précédents modes de réalisation. Ainsi, dans le cas présent, seulement deux oeillets 611 et 612 sont agencés dans la paroi transverse 605, et de ce fait l'implant 600 comprend seulement deux chemins de suture. Les deux oeillets 611 et 613 sont décalés axialement dans la paroi transverse 605. Chaque chemin de suture est débouchant vers l'extrémité proximale 621 et non débouchant vers l'extrémité distale 625. Deux fils de suture, non représentés, peuvent alors former chacun un arc dans l'oeillet respectif 611 ou 612, et s'étendre à l'extérieur du corps 602 en passant par la cavité transversale 604 et la cavité proximale 603. Les axes Y611 et Y612 des deux oeillets 611 et 612 sont sensiblement parallèles entre eux, et perpendiculaires et sécants avec l'axe longitudinal X-X.

Dans ce sixième mode de réalisation, les oeillets 611 et 612 délimitent l'espace transversal 680, tandis que les parties latérales 681 et 682 des chemins de suture sont situées respectivement du côté Z1 et du côté Z2, débouchantes vers l'extrémité proximale 621 et non débouchantes vers l'extrémité distale 625.

Selon une variante non représentée, la réduction de diamètre dans la partie distale 624 est moins prononcée, et l'extrémité distale 625 du corps 602 ne présente pas une pointe distincte de la partie distale 604.

Selon une autre variante non représentée, la cavité proximale 603 peut comprendre deux portions distinctes, agencées de manière analogues à l'un des implants 300, 400 et 500 et adaptées pour l'agencement dans l'implant 600 de seulement deux oeillets 611 et 612 et deux fils de suture.

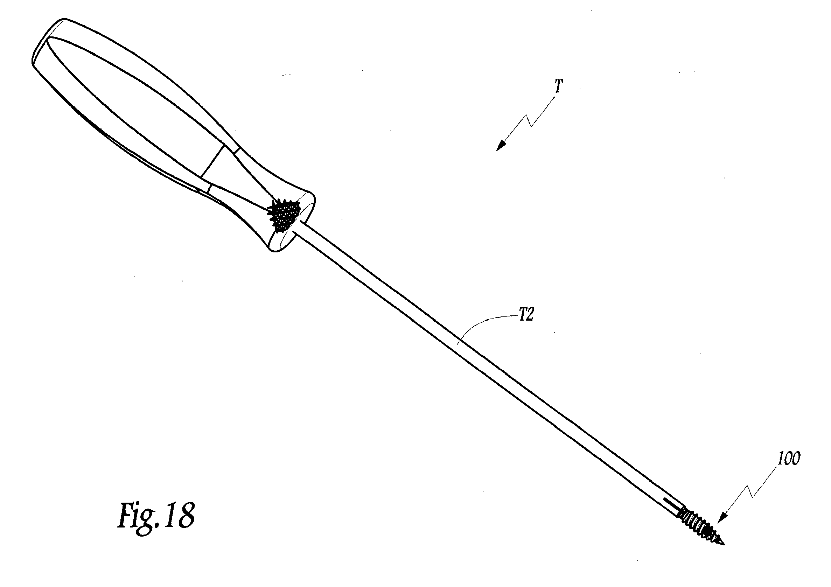

Sur la figure 18 est représenté l'implant de suture 100 et un outil T permettant la mise en place de l'implant de suture 100 dans l'os du patient, non représenté.

En pratique, une tige longitudinale T2 de l'outil T vient se loger dans la cavité proximale 103 du corps 102 de l'implant 100, afin de pouvoir lui transmettre un mouvement de rotation et un couple. En particulier, la pointe de la tige T2 est adaptée pour être insérée dans une cavité 103 présentant une section hexagonale et/ou carrée. Les fils de suture 161, 162 et 163 sont positionnés dans l'implant 100 avant que ce dernier ne soit positionné à l'extrémité de la tige T2 de l'outil T. De préférence, comme la surface extérieure 120 du corps 102 de l'implant 100 est filetée, l'outil T est un tournevis.

En variante non représentée, l'outil peut être de tout type adapté à la mise en place de l'implant de suture dans l'os du patient, en lui imprimant un mouvement de rotation. Un tel outil est généralement adapté pour un unique diamètre d'implant, mais il peut être avantageusement adapté à tout type d'implant, de diamètre variable.

De préférence, les implants 100, 200, 300, 400, 500 et 600 sont réalisés en matériau polymère, notamment polyétheréthercétone (PEEK), ou en matériau métallique, notamment titane. Selon la résistance du matériau et la configuration en pointe ou non, l'implant peut être disposé dans une cavité osseuse préparée avant son insertion, ou bien être autotaraudeur.

De préférence, le corps des implants est monobloc, ce qui facilite leur fabrication.

En variante, certains éléments pourraient être rapportés ou surmoulés dans le corps d'implant. Dans ce cas, on s'assure que l'implant ne présente aucune arête vive susceptible d'endommager les fils de suture.

Selon une autre variante, l'implant n'est que partiellement fileté.

Selon une autre variante, l'implant peut comprendre plus de trois oeillets de passages d'autant de fils de suture, agencés de différentes manières dans le corps de l'implant, dans la paroi transverse. Le cas échéant, la configuration selon l'un ou l'autre mode de réalisation peut être adaptée au nombre d'oeillets et à leur agencement. Chaque fil de suture dispose de son propre chemin de suture.

Selon une variante particulière de l'implant 400, les parois 405 et 435 peuvent être confondues, et comportent des oeillets 411, 412 et/ou 413. De même pour l'implant 500, les parois 505 et 535 peuvent être formées de manière monobloc et présenter une configuration similaire à la variante précitée de l'implant 400.

De manière générale, quel que soit le mode de réalisation, les chemins de suture sont délimités, d'une part, par la ou les parois transverses et les oeillets et, d'autre part, par la cavité transversale et la cavité proximale. Aucun implant de suture existant ne protège les fils de suture avec une efficacité comparable, tout en procurant une manipulation aisée et un glissement indépendant de ces fils.

Selon l'invention, les chemins de suture s'étendent de part et d'autre de la paroi transverse, sont débouchants vers l'extrémité proximale et non débouchants vers l'extrémité distale. L'espace transversal est apte à recevoir au moins un fil de suture, est situé dans la partie courante du corps, et traverse le plan de référence Pz. En effet, chaque implant comporte au moins une paroi transverse qui s'étend sensiblement dans le plan Pz et qui est agencée au moins partiellement dans la cavité transversale et/ou la cavité proximale. De préférence, cette paroi transverse est la paroi 105, 205, 305, 405, 505 ou 605 précédemment définie, comportant des oeillets. Différentes configurations de l'implant de suture, notamment de la ou les parois transverses et de l'espace transversal, peuvent être envisagées sans sortir du cadre de l'invention.

Ainsi, les chemins de suture présentent une géométrie et sont positionnés dans le corps de l'implant de telle sorte qu'un fil de suture en place dans l'implant forme un U avec un premier brin rectiligne qui est engagé à la fois dans la cavité proximale et dans la cavité transversale, sur un premier côté Z1 du plan de référence Pz, un second brin rectiligne qui est engagé à la fois dans la cavité proximale et dans la cavité transversale, sur un second côté Z2 du plan de référence Pz, en étant sensiblement parallèle au premier brin, et un arc qui est engagé dans l'espace transversal et qui relie le premier brin et le second brin, du premier côté Z1 vers le deuxième côté Z2 du plan de référence Pz.

Les caractéristiques techniques des différents modes de réalisation peuvent être, en totalité ou pour certaines d'entre elles, combinées entre elles. Ainsi, l'implant peut être adapté à une application particulière, notamment en termes de coût, de simplicité d'utilisation et de performance.

Durant la réparation d'un tissu mou, le risque d'endommagement contre les fils de suture ou une partie tranchante ou pointue de l'implant est fortement limité. L'implant est simple et peu coûteux à fabriquer et présente un encombrement réduit. De plus, le filetage offre une bonne résistance à la tension de retrait. Ainsi, l'implant de suture selon l'invention peut être ancré de manière solide dans la masse osseuse du patient, ce qui favorise la guérison et offre une assurance supplémentaire au chirurgien.

## Revendications

1. Implant de suture (100), comprenant :
- un corps allongé (102) présentant une extrémité proximale (121) et une extrémité distale (125), le corps incluant au moins deux chemins de suture (180, 181, 182) qui sont débouchants vers l'extrémité proximale et non débouchants vers l'extrémité distale et qui sont respectivement aptes à recevoir un fil de suture (161, 162, 163),
- une cavité proximale (103) incluse dans le corps et qui s'étend selon un axe longitudinal (X-X) du corps, et
- une cavité transversale (104) qui s'étend selon un premier axe (Y-Y) transversal à l'axe longitudinal (X-X) et dans laquelle la cavité proximale débouche,
- une paroi transverse (105) qui est agencée au moins partiellement dans la cavité transversale (104)
- pour chaque chemin de suture (180, 181, 182), un oeillet distinct (111, 112, 113) de passage de fil de suture (161, 162, 163), qui traverse la paroi transverse (105) en s'étendant sensiblement selon un axe (Y111, Y112, Y113) parallèle au premier axe transversal (Y-Y), de sorte qu'un fil de suture en place dans l'implant (100) forme un U avec :
- un premier brin rectiligne (161 a, 162a, 163a) s'étendant sensiblement parallèle à l'axe longitudinal (X-X),
- un second brin rectiligne (161 c, 162c, 163c) sensiblement parallèle au premier brin, et
- un arc (161 b, 162b, 163b) formé dans l'oeillet et reliant le premier brin et le second brin,
**caractérisé en ce que**:
l'arc est formé dans l'oeillet et
la paroi transverse s'étend essentiellement dans un plan (Pz) comprenant l'axe longitudinal (X-X) et un second axe (Z-Z) transversal à l'axe longitudinal (X-X), la paroi transverse (105) présentant deux faces (157, 158) sensiblement planes, parallèles entre elles et perpendiculaires au premier axe (Y-Y), les brins rectilignes s'étendant en partie le long des deux faces.

2. Implant (100) selon l'une des revendications précédentes, **caractérisé en ce que** la paroi transverse (105) s'étend en direction de la cavité proximale (103) depuis une face distale (141) de la cavité transversale (104).

3. Implant (100) selon l'une des revendications précédentes, **caractérisé en ce que** la paroi transverse (105) s'étend partiellement à l'intérieur de la cavité proximale.

4. Implant (100) selon l'une des revendications précédentes, **caractérisé en ce qu'**au moins deux des oeillets (111, 112, 113) sont décalés dans la paroi transverse (105) selon la direction de l'axe longitudinal (X-X).

5. Implant (100) selon l'une des revendications précédentes, **caractérisé en ce qu'**au moins deux des oeillets (111, 112, 113) sont décalés dans la paroi transverse (105) selon la direction du second axe (Z-Z).

6. Implant (100) selon l'une des revendications précédentes, **caractérisé en ce que** le corps (102) inclut trois chemins de suture (180, 181, 182) qui sont débouchants vers l'extrémité proximale (121) et non débouchants vers l'extrémité distale (125).

7. Implant (100) selon la revendication 6, **caractérisé en ce que** les axes (Y111, Y112, Y113) des trois oeillets (111, 112, 113) associés aux trois chemins de suture (180, 181, 182) définissent un triangle isocèle, dans un plan (Pz) comprenant l'axe longitudinal (X-X) et le second axe (Z-Z).

8. Implant (100) selon l'une des revendications précédentes, **caractérisé en ce que** la cavité proximale (103) comprend une première portion de cavité (131) qui présente une section transversale de forme hexagonale.

9. Implant (400) selon la revendication 8, **caractérisé en ce que** la cavité proximale (403) comprend en outre une deuxième portion de cavité (432) avec deux orifices (432a, 432c) qui présentent chacun une section transversale de forme sensiblement rectangulaire, et dont les parois longitudinales de plus grandes dimensions sont sensiblement parallèles aux faces longitudinales correspondantes de la paroi transverse (405).

10. Implant (500) selon la revendication 8, **caractérisé en ce que** la cavité proximale (503) comprend en outre une deuxième portion de cavité (532) avec, pour chaque oeillet (511, 512, 513), deux orifices (571 a, 571 c, 572a, 572b, 573a, 573c) qui sont agencés symétriquement par rapport au plan (Pz) comprenant l'axe longitudinal (X-X) et le second axe transversal (Z-Z), qui présentent chacun une section transversale de forme sensiblement circulaire, l'axe de chacun des orifices étant parallèle à l'axe longitudinal (X-X) du corps et perpendiculaire au premier axe transversal (Y-Y), en intersectant l'axe de l'oeillet correspondant (Y511, Y512, Y513).

11. Implant (100) selon l'une des revendications précédentes, **caractérisé en ce que** chaque oeillet (111, 112, 113) présente des parois de forme sensiblement parabolique en section dans le plan (Py) comprenant l'axe longitudinal (X-X) et le premier axe transversal (Y-Y).

12. Implant (100) selon l'une des revendications précédentes, **caractérisé en ce que** le corps (102) est monobloc.

13. Implant selon l'une des revendications 1 à 12, **caractérisé en ce que** le corps est en matériau polymère, notamment polyétheréthercétone (PEEK).

14. Implant selon l'une des revendications 1 à 12, **caractérisé en ce que** le corps est en matériau métallique, notamment titane (Ti).

## Patentansprüche

1. Naht-Implantat (100), umfassend:
- einen längsgerichteten Körper (102), der ein proximales Ende (121) und ein distales Ende (125) aufweist, wobei der Körper mindestens zwei Nahtwege (180, 181, 182) einschließt, die zu dem proximalen Ende und nicht zu dem distalen Ende münden und die jeweils geeignet sind, ein Nahtfaden (161, 162, 163) aufzunehmen,
- einen proximalen Hohlraum (103), der in dem Körper eingeschlossen ist und sich gemäß einer Längsachse (X-X) des Körpers erstreckt, und
- einen Querhohlraum (104), der sich gemäß einer ersten Achse (Y-Y) quer zur Längsachse (X-X) erstreckt und in den der proximale Hohlraum mündet,
- eine Querwand (105), die mindestens teilweise in dem Querhohlraum (104) angeordnet ist
- für jeden Nahtweg (180, 181, 182) ein separates Öhr (111, 112, 113) für den Durchgang des Nahtfadens (161, 162, 163), das die Querwand (105) durchquert, indem es sich im Wesentlichen gemäß einer Achse (Y111, Y112, Y113) parallel zur ersten Querachse (Y-Y) erstreckt, derart, dass ein Nahtfaden, der in dem Implantat (100) in Stellung ist, ein U mit bildet mit:
- einem ersten geraden Fadenstrang (161a, 162a, 163a), der sich im Wesentlichen parallel zur Längsachse (X-X) erstreckt,
- einem zweiten Fadenstrang (161c, 162c, 163c), der sich im Wesentlichen parallel zum ersten Fadenstrang erstreckt und
- einem Bogen (161b, 162b, 163b), der in dem Öhr gebildet wird und den ersten Fadenstrang mit dem zweiten Fadenstrang verbindet,
**dadurch gekennzeichnet, dass**:
der Bogen in dem Öhr gebildet wird und
die Querwand sich im Wesentlichen in einer Ebene (Pz) erstreckt, die die Längsachse (X-X) und eine zweite Achse (Z-Z) quer zur Längsachse (X-X) umfasst, wobei die Querwand (105) zwei im Wesentlichen ebene Flächen (157, 158), die untereinander parallel sind und senkrecht zur ersten Achse (Y-Y) liegen, aufweist, wobei die geradlinigen Fadenstränge sich teilweise längs der zwei Flächen erstrecken.

2. Implantat (100) nach einem der vorhergehenden Ansprüche, **dadurch gekennzeichnet, dass** die Querwand (105) sich in Richtung des proximalen Hohlraums (103) von einer distalen Fläche (141) des Querhohlraums (104) erstreckt.

3. Implantat (100) nach einem der vorhergehenden Ansprüche, **dadurch gekennzeichnet, dass** die Querwand (105) sich teilweise im Inneren des proximalen Hohlraums erstreckt.

4. Implantat (100) nach einem der vorhergehenden Ansprüche, **dadurch gekennzeichnet, dass** mindestens zwei der Öhre (111, 112, 113) in der Querwand (105) gemäß der Richtung der Längsachse (X-X) versetzt sind.

5. Implantat (100) nach einem der vorhergehenden Ansprüche, **dadurch gekennzeichnet, dass** mindestens zwei der Öhre (111, 112, 113) in der Querwand (105) gemäß der Richtung der zweiten Achse (Z-Z) versetzt sind.

6. Implantat (100) nach einem der vorhergehenden Ansprüche, **dadurch gekennzeichnet, dass** der Körper (102) drei Nahtwege (180, 181, 182) einschließt, die zu dem proximalen Ende (121) und nicht zu dem distalen Ende (125) münden.

7. Implantat (100) nach Anspruch 6, **dadurch gekennzeichnet, dass** die Achsen (Y111, Y112, Y113) der drei Öhre (111, 112, 113), die den drei Nahtwegen (180, 181, 182) zugeordnet sind, ein gleichschenkliges Dreieck in einer Ebene (Pz) bilden, die die Längsachse (X-X) und die zweite Achse (Z-Z) umfasst.

8. Implantat (100) nach einem der vorhergehenden Ansprüche, **dadurch gekennzeichnet, dass** der proximale Hohlraum (103) einen ersten Hohlraumbereich (130) umfasst, der einen Querschnitt in hexagonaler Form aufweist.

9. Implantat (100) nach Anspruch 8, **dadurch gekennzeichnet, dass** der proximale Hohlraum (403) außerdem zwei Hohlraumbereiche (432) mit zwei Öffnungen (432a, 432c) umfasst, die jeweils einen Querschnitt in im Wesentlichen rechteckiger Form aufweisen und deren Längswände mit den größten Abmessungen im Wesentlichen parallel zu den korrespondierenden Längsflächen der Querwand (405) ausgebildet sind.

10. Implantat (500) nach Anspruch 8, **dadurch gekennzeichnet, dass** der proximale Hohlraum (503) außerdem einen zweiten Hohlraumbereich (532) mit zwei Öffnungen (571a, 571c, 572a, 572b, 573a, 573c) für jedes Öhr (511, 512, 513) umfasst, die symmetrisch in Bezug auf die die Längsachse (X-X) und die zweite Querachse (Z-Z) umfassende Ebene (Pz) angeordnet sind, die jeweils einen Querschnitt in im Wesentlichen kreisförmiger Form aufweisen, wobei die Achse jeder der Öffnungen parallel zur Längsachse (X-X) des Körpers und senkrecht zur ersten Querachse (Y-Y) liegt, wobei sie die Achse des korrespondierenden Öhrs (Y511, Y512, Y513) schneidet.

11. Implantat (100) nach einem der vorhergehenden Ansprüche, **dadurch gekennzeichnet, dass** jedes Öhr (111, 112, 113) Wände in im Wesentlichen parabolischer Form im Schnitt in der Ebene (Py) aufweist, die die Längsachse (X-X) und die zweite Querachse (Y-Y) umfasst.

12. Implantat (100) nach einem der vorhergehenden Ansprüche, **dadurch gekennzeichnet, dass** der Körper (102) einstückig ist.

13. Implantat nach einem der Ansprüche 1 bis 12, **dadurch gekennzeichnet, dass** der Körper aus einem Polymermaterial, insbesondere Polyetheretherketon (PEEK) besteht.

14. Implantat nach einem der Ansprüche 1 bis 12, **dadurch gekennzeichnet, dass** der Körper aus Metall, insbesondere Titan (Ti) besteht.

## Claims

1. Suture anchor (100), comprising:
- an elongated body (102) having a proximal end (121) and a distal end (125), the body including at least two suture paths (180, 181, 182) which are open towards the proximal end and not open towards the distal end and which are respectively capable of receiving a suture thread (161, 162, 163),
- a proximal cavity (103) which is included in the body and which extends on a longitudinal axis (X-X) of the body, and
- a transverse cavity (104) which extends on a first axis (Y-Y) that is transversal to the longitudinal axis (X-X) and into which the proximal cavity opens,
- a transverse wall (105) which is arranged at least partially in the transverse cavity (104), and
- for each suture path (180, 181, 182), a distinct eyelet (111, 112, 113) for the passage of the suture thread (161, 162, 163), which passes through the transverse wall (105) while extending substantially on an axis (Y111, Y112, Y113) parallel to the first transverse axis (Y-Y), so that a suture thread in place in the anchor (100) forms a U with:
- a first rectilinear strand (161a, 162a, 163a) extending substantially on the longitudinal axis (X-X),
- a second rectilinear strand (161c, 162c, 163c) substantially parallel to the first strand, and
- an arc (161 b, 162b, 163b) formed in the eyelet and connecting the first strand and the second strand.
**characterized in that**:
the arc is formed in the eyelet and
the transverse wall (105) extends essentially in a plane (Pz) comprising the longitudinal axis (X-X) and a second axis (Z-Z) that is transversal to the longitudinal axis (X-X), the transverse wall (105) having two substantially flat faces (157, 158) parallel with one another and perpendicular to the first axis (Y-Y), the rectilinear strands extending partly along these two faces.

2. Anchor (100) according to one of the preceding claims, **characterized in that** the transverse wall (105) extends in the direction of the proximal cavity (103) from a distal face (141) of the transverse cavity (104).

3. Anchor (100) according to one of the preceding claims, **characterized in that** the transverse wall (105) extends partially inside the proximal cavity.

4. Anchor (100) according to one of the preceding claims, **characterized in that** at least two of the eyelets (111, 112, 113) are offset in the transverse wall (105) in the direction of the longitudinal axis (X-X).

5. Anchor (100) according to one of the preceding claims, **characterized in that** at least two of the eyelets (111, 112, 113) are offset in the transverse wall (105) in the direction of the second axis (Z-Z).

6. Anchor (100) according to one of the preceding claims, **characterized in that** the body (102) includes three suture paths (180, 181, 182) which are open towards the proximal end (121) and not open towards the distal end (125).

7. Anchor (100) according to Claim 6, **characterized in that** the axes (Y111, Y112, Y113) of the three eyelets (111, 112, 113) associated with the three suture paths (180, 181, 182) define an isosceles triangle, in a plane (Pz) comprising the longitudinal axis (X-X) and the second axis (Z-Z).

8. Anchor (100) according to one of the preceding claims, **characterized in that** the proximal cavity (103) comprises a first cavity portion (131) which has a hexagonal-shaped cross section.

9. Anchor (400) according to Claim 8, **characterized in that** the proximal cavity (403) also comprises a second cavity portion (432) with two orifices (432a, 432c) which each have a substantially rectangular cross section and of which the longitudinal walls with the largest dimensions are substantially parallel to the corresponding longitudinal faces of the transverse wall (405).

10. Anchor (500) according to Claim 8, **characterized in that** the proximal cavity (503) also comprises a second cavity portion (532) with, for each eyelet (511, 512, 513), two orifices (571 a, 571c, 572a, 572b, 573a, 573c) which are arranged symmetrically relative to the plane (Pz) comprising the longitudinal axis (X-X) and the second transverse axis (Z-Z), which each have a substantially circular cross section, the axis of each of the orifices being parallel to the longitudinal axis (X-X) of the body and perpendicular to the first transverse axis (Y-Y), while intersecting the axis of the corresponding eyelet (Y511, Y512, Y513).

11. Anchor (100) according to one of the preceding claims, **characterized in that** each eyelet (111, 112, 113) has substantially parabola-shaped walls in section in the plane (Py) comprising the longitudinal axis (X-X) and the first transverse axis (Y-Y).

12. Anchor (100) according to one of the preceding claims, **characterized in that** the body (102) is in one piece.

13. Anchor according to one of Claims 1 to 12, **characterized in that** the body is made of a polymer, notably polyether ether ketone (PEEK).

14. Anchor according to one of Claims 1 to 12, **characterized in that** the body is made of metal, notably titanium (Ti).
